# EUROPÄISCHE PATENTSCHRIFT

(11) **EP 2 859 341 B1**
(45) Veröffentlichungstag und Bekanntmachung des Hinweises auf die Patenterteilung: **23.11.2016**
(21) Anmeldenummer: 13732803.5
(22) Anmeldetag: 07.06.2013
(51) Int. Cl.: G01N 33/36

(54) **VERGLEICH DER QUALITÄTEN VON LÄNGLICHEN TEXTILEN PRÜFGÜTERN**
COMPARING THE QUALITY OF ELONGATE TEXTILE SAMPLES
COMPARAISON DES QUALITÉS D'ÉCHANTILLONS TEXTILES ALLONGÉS

(30) Priorität: 11.06.2012 CH 807122012
(43) Veröffentlichungstag der Anmeldung: 15.04.2015
(73) Patentinhaber: Uster Technologies AG, 8610 Uster (CH)
(72) Erfinder: NARAYANAN, Sivakumar, CH-8610 Uster (CH); SCHMID, Peter, CH-8050 Zürich (CH)
(74) Vertreter: Pliska, Pavel
(86) Internationale Anmeldenummer: PCT/CH2013/000097
(87) Internationale Veröffentlichungsnummer: WO 2013/185246

(56) Entgegenhaltungen:
- EP-A1- 0 685 580
- WO-A1-2010/078665
- WO-A1-2012/122663

## Beschreibung

### FACHGEBIET

Die vorliegende Erfindung liegt auf dem Gebiet der textilen Qualitätskontrolle. Sie betrifft ein Verfahren und eine Vorrichtung zum Vergleich der Qualitäten von länglichen textilen Prüfgütern, gemäss den Oberbegriffen der unabhängigen Patentansprüche.

### STAND DER TECHNIK

Es ist eine Vielzahl verschiedenartiger Vorrichtungen zur Untersuchung oder Prüfung von Garn bekannt. Sie lassen sich nach ihrer Anwendung in die beiden Kategorien Laborprüfung (offline) und Prüfung während des Produktionsprozesses (online) einteilen. Die Anlage USTER^{®} *CLASSIMAT QUANTUM* der Anmelderin, wie sie z. B. im Anwendungshandbuch "USTER® CLASSIMAT QUANTUM", Uster Technologies AG, Mai 2005, beschrieben ist, dient der Klassierung von Garnfehlern wie Dick- und Dünnstellen sowie Fremdstoffen. Der Funktion nach ist sie ein Laborgerät, weil sie im Textillabor zur detaillierten Prüfung von Stichproben verwendet wird. Die Geräte, aus denen sie besteht, stammen jedoch grösstenteils aus der Produktion: Das untersuchte Garn wird auf einer Spulstelle einer manuellen Spulmaschine umgespult und mit einem Garnreinigermesskopf abgetastet. Die vom Garnreinigermesskopf gemessenen Garnparameter werden in einer Steuereinheit und/oder einem Arbeitsplatzrechner statistisch ausgewertet, z. B. in einem zweidimensionalen Klassierschema klassiert.

Das zweidimensionale Klassierschema, das auch Ereignisfeld genannt werden kann, wird üblicherweise durch ein kartesisches Koordinatensystem mit einer Abszisse und einer Ordinate aufgespannt. Entlang der Abszisse ist die Fehlerlänge und entlang der Ordinate die Fehleramplitude (Abweichung der Masse pro Garnlänge, des Garndurchmessers, der Garnreflektivität etc. von einem Sollwert) aufgetragen. Jede dieser Achsen kann in Abschnitte unterteilt werden, wodurch ein System von rechteckigen Klassen von Garnfehlern entsteht. Im System USTER^{®} *CLASSIMAT QUANTUM* sind es 23 oder 27 Klassen. Während der Garnprüfung werden die festgestellten Garnfehler in die entsprechenden Klassen klassiert, und die Anzahl der festgestellten Fehler wird für jede Klasse einzeln angezeigt. Das so vervollständigte Klassierschema gibt einerseits einen Eindruck von der Qualität des Garns und ermöglicht andererseits eine quantitative Grundlage für die Festlegung der Reinigungsgrenze für das Garn. Beispiele für derartige Klassierschemata finden sich im erwähnten Anwendungshandbuch "USTER^{®} *CLASSIMAT QUANTUM"* und in der Patentschrift US-5,537,811 A. Die US-6,374,152 B1 zeigt ein Klassierschema, in das zusätzlich eine Punktewolke, deren Punkte die festgestellten Garnfehler darstellen, und eine Reinigungsgrenze für die Garnfehler eingezeichnet sind. Die US-6,244,030 B1 gibt ein Beispiel für ein Klassierschema an, entlang dessen Ordinate die Reflektivität des Garns aufgetragen ist.

Garnreiniger werden zur Sicherung der Garnqualität an Spinn- oder Spulmaschinen eingesetzt. Ziel der Garnreinigung ist es, Fehlstellen wie Dickstellen, Dünnstellen oder Fremdstoffe im Garn zu detektieren, gemäss bestimmten Qualitätskriterien zu bewerten und gegebenenfalls zu eliminieren. Zu diesem Zweck beinhaltet ein Garnreinigermesskopf einen Messschlitz, durch den das Garn entlang seiner Längsrichtung hindurchläuft. Entlang des Messschlitzes ist mindestens ein Sensor zum Abtasten des bewegten Garns angeordnet. Häufig verwendete Sensorprinzipien sind das kapazitive (siehe z. B. EP-0'924'513 A1) oder das optische (siehe z. B. WO-93/13407 A1). Ferner beinhaltet der Garnreinigermesskopf eine elektronische Schaltung zur Auswertung des Sensorsignals und zum Vergleich des Signals mit vorgegebenen Qualitätskriterien, z. B. einer Reinigungsgrenze. Liegt eine Fehlstelle unterhalb der Reinigungsgrenze, so ist sie tolerierbar; liegt sie oberhalb der Reinigungsgrenze, so ist sie nicht tolerierbar und wird aus dem Garn entfernt oder zumindest registriert.

Die WO-2010/078665 A1 beschreibt ein Verfahren und eine Vorrichtung zur Charakterisierung eines entlang seiner Längsrichtung bewegten textilen Prüfgutes. Dabei werden Messwerte einer Eigenschaft des Prüfgutes entlang seiner Längsrichtung erfasst. Aus den Messwerten werden Werte eines Prüfgutparameters ermittelt. Aus den Werten des Prüfgutparameters und ihrer Erstreckung in der Längsrichtung werden Dichten von Ereignissen in dem Ereignisfeld ermittelt. In dem Ereignisfeld wird ein Prüfgutkörper als Fläche grafisch dargestellt. Die Fläche wird einerseits durch die Abszisse, andererseits durch die Ordinate und ferner durch eine Linie in dem Ereignisfeld, welche im Wesentlichen einer konstanten Ereignisdichte folgt, begrenzt. Die Darstellung des Prüfgutkörpers versetzt eine Bedienungsperson in die Lage, charakteristische Eigenschaften des Prüfgutes schnell zu erfassen und eine Reinigungsgrenze rationell vorzugeben.

Die US-6,343,508 B1 stellt sich die Aufgabe, Werte von Parametern eines Garns auf einen Blick erfassbar zu machen und dabei die Parameter gemäss ihrer Wichtigkeit zu gewichten. Zu diesem Zweck werden die verschiedenen Garnparameter in einem Segmentdiagramm dargestellt, wobei jedem Parameter ein Segment zugeordnet ist. Die Gewichtung erfolgt mittels unterschiedlicher Öffnungswinkel der einzelnen Segmente. Zum Bewertung des jeweiligen Garns können in das Segmentdiagramm auch entsprechende Referenzwerte für die Parameter eingezeichnet werden. Die Referenzwerte können z. B. den USTER^{®} *STATISTICS* entnommen werden. Die USTER^{®} *STATISTICS* sind eine von der Anmelderin des vorliegenden Schutzrechtes herausgegebene Zusammenstellung von textilen Qualitätsdaten, die aus der weltweiten Produktion von textilen Rohmaterialien, Zwischenprodukten und Endprodukten ermittelt wurden; siehe CD-ROM "USTER® STATISTICS 2007", Version 4.0, Uster Technologies AG, 2011.

### DARSTELLUNG DER ERFINDUNG

Es ist eine Aufgabe der vorliegenden Erfindung, ein Verfahren und eine Vorrichtung anzugeben, die einen einfachen Vergleich der Qualitäten von länglichen textilen Prüfgütern erlauben. Unter "Qualität" werden hier Merkmale wie Massenschwankungen entlang der Längsrichtung, Durchmesserschwankungen entlang der Längsrichtung, Fremdstoffgehalt, Haarigkeit etc. verstanden.

Diese und andere Aufgaben werden durch das erfindungsgemässe Verfahren und die erfindungsgemässe Vorrichtung, wie sie in den unabhängigen Patentansprüchen definiert sind, gelöst. Vorteilhafte Ausführungsformen sind in den abhängigen Patentansprüchen angegeben.

Im erfindungsgemässen Verfahren zum Vergleich der Qualitäten von länglichen textilen Prüfgütern werden Messwerte mindestens einer Eigenschaft eines ersten Prüfgutes entlang der Längsrichtung des ersten Prüfgutes erfasst. Werte mindestens eines Parameters des ersten Prüfgutes werden aus den Messwerten ermittelt. Ein Ereignisfeld wird bereitgestellt, das einen Quadranten oder einen Teil eines Quadranten eines zweidimensionalen kartesischen Koordinatensystems beinhaltet, dessen Abszisse eine Erstreckung von Parameterwerten in der Längsrichtung und dessen Ordinate eine Abweichung der Parameterwerte von einem Sollwert angibt, so dass die ermittelten Abweichungen der Parameterwerte und ihre Erstreckungen in der Längsrichtung als Ereignisse in dem Ereignisfeld eingetragen werden. Es werden Dichten der Ereignisse in dem Ereignisfeld ermittelt. In dem Ereignisfeld wird eine erste Dichtelinie, welche im Wesentlichen einer konstanten Dichte der am ersten Prüfgut ermittelten Ereignisse folgt, dargestellt. In dem Ereignisfeld wird zusätzlich zur ersten Dichtelinie eine Referenzdichtelinie, welche zwar derselben Ereignisdichte wie die erste Dichtelinie folgt, sich aber auf ein vom ersten Prüfgut verschiedenes Referenzprüfgut bezieht, dargestellt.

Der Parameter des Prüfgutes kann z. B. eine Masse pro Längeneinheit, eine Querdimension oder ein Fremdstoffgehalt des Prüfgutes sein.

Das Referenzprüfgut kann ein zweites, vom ersten Prüfgut verschiedenes reales Prüfgut sein. Diesfalls wird die Referenzdichtelinie auf dieselbe Weise wie die erste Dichtelinie ermittelt. Ein reales Referenzprüfgut wird entlang seiner Längsrichtung ausgemessen, Werte des mindestens einen Parameters werden aus den Messwerten ermittelt, Ereignisdichten werden aus den Parameterwerten ermittelt, und die Referenzdichtelinie folgt im Wesentlichen einer konstanten Dichte der am Referenzprüfgut ermittelten Ereignisse.

Alternativ kann das Referenzprüfgut ein virtuelles Prüfgut sein, das zum Zwecke der Bestimmung der Referenzdichtelinie gebildet wird. Das virtuelle Referenzprüfgut kann aus einer Menge von mindestens zwei realen Prüfgütern gebildet werden. Die Menge kann nebst mindestens einem anderen realen Prüfgut auch das erste Prüfgut beinhalten. Es werden mindestens zwei reale Prüfgüter ausgemessen, und die Referenzdichtelinie wird als Mittelwert aller ermittelten Dichtelinien oder einer Teilmenge davon definiert. Bei der Mittelwertbildung kann eine Gewichtung vorgenommen werden, bspw. anhand der jeweils erfassten Länge des betreffenden Prüfgutes. Die Referenzdichtelinie braucht nicht unmittelbar vor der Ausmessung des ersten Prüfgutes ermittelt worden zu sein. Sie kann einem Qualitäts-Referenzwerk entnommen werden, wie z. B. den USTER^{®} *STATISTICS* der Anmelderin des vorliegenden Schutzrechtes.

In einer bevorzugten Ausführungsform wird eine zumindest teilweise von der ersten Dichtelinie und der Referenzdichtelinie begrenzte Differenzfläche in dem Ereignisfeld grafisch hervorgehoben. Die Hervorhebung kann dadurch erfolgen, das sich die Differenzfläche grafisch von ihrer Umgebung unterscheidet, insbesondere indem sie eine andere Farbe, einen anderen Grauton und/oder ein anderes Muster aufweist als ihre Umgebung.

In dem Ereignisfeld kann ein Referenzprüfgutkörper als Fläche dargestellt werden, die einerseits durch die Abszisse oder eine parallel dazu verlaufende Gerade, andererseits durch die Ordinate oder eine parallel dazu verlaufende Gerade und ferner durch die Referenzdichtelinie begrenzt wird. Die den Referenzprüfgutkörper darstellende Fläche kann sich grafisch von ihrer Umgebung unterscheiden, insbesondere indem sie eine andere Farbe, einen anderen Grauton und/oder ein anderes Muster aufweist als ihre Umgebung. Ein Prüfgutkörper an sich ist aus der WO-2010/078665 A1 bekannt.

Vorteilhaft ist es, wenn die konstante Ereignisdichte zwischen 500 und 2000 Ereignissen pro 100 km Prüfgutlänge und vorzugsweise bei 1000 Ereignissen pro 100 km Prüfgutlänge liegt.

Zumindest ein Teil des Ereignisfeldes kann durch waagrechte Klassengrenzen und senkrechte Klassengrenzen in rechteckige Klassen für Ereignisse unterteilt werden.

Die Erfindung ermöglicht es, in dem Ereignisfeld zusätzlich zur sich auf das Referenzprüfgut beziehenden Referenzdichtelinie und zur sich auf das erste Prüfgut beziehenden ersten Dichtelinie mindestens eine sich auf ein zweites Prüfgut beziehende zweite Dichtelinie darzustellen. Vorzugsweise unterscheiden sich die erste Dichtelinie, die Referenzdichtelinie und allenfalls die zusätzlich dargestellte mindestens eine zweite Dichtelinie grafisch voneinander, insbesondere indem sie unterschiedliche Farben, unterschiedliche Grautöne, unterschiedliche Dicken und/oder unterschiedliche Strichsymbole aufweisen.

Das erfindungsgemässe Verfahren wird vorzugsweise von einem Computer ausgeführt.

Die erfindungsgemässe Vorrichtung zum Vergleich der Qualität von länglichen textilen Prüfgütern beinhaltet eine Messeinheit zur Erfassung von Messwerten mindestens einer Eigenschaft eines ersten Prüfgutes entlang der Längsrichtung des ersten Prüfgutes. Sie beinhaltet ferner eine mit der Messeinheit verbundene Auswerteeinheit, welche für die Ermittlung von Werten mindestens eines Parameters des ersten Prüfgutes aus den Messwerten, für die Bereitstellung eines Ereignisfeldes, das einen Quadranten oder einen Teil eines Quadranten eines zweidimensionalen kartesischen Koordinatensystems beinhaltet, dessen Abszisse eine Erstreckung von Parameterwerten in der Längsrichtung und dessen Ordinate eine Abweichung der Parameterwerte von einem Sollwert angibt, so dass die ermittelten Abweichungen der Parameterwerte und ihre Erstreckungen in der Längsrichtung als Ereignisse in dem Ereignisfeld eintragbar sind, und für die Ermittlung von Dichten der Ereignisse in dem Ereignisfeld eingerichtet ist. Ausserdem beinhaltet die Vorrichtung eine mit der Auswerteeinheit verbundene Ausgabeeinheit zur Darstellung des Ereignisfeldes und einer ersten Dichtelinie in dem Ereignisfeld, welche im Wesentlichen einer konstanten Dichte der am ersten Prüfgut ermittelten Ereignisse folgt. Die Auswerteeinheit ist zur Speicherung einer Referenzdichtelinie, welche zwar derselben Ereignisdichte wie die erste Dichtelinie folgt, sich aber auf ein vom ersten Prüfgut verschiedenes Referenzprüfgut bezieht, eingerichtet. Die Ausgabeeinheit ist zur gleichzeitigen Darstellung der ersten Dichtelinie und der Referenzdichtelinie eingerichtet. Anhand dieser Darstellung kann ein Vergleich der Verläufe der ersten Dichtelinie und der Referenzdichtelinie vorgenommen werden.

Der Begriff "Auswerteeinheit" bezeichnet in dieser Schrift eine funktionale und nicht unbedingt eine physische Einheit. Die Auswerteeinheit kann ein, zwei oder mehrere physische Geräte umfassen, welche die beschrieben Auswertefunktionen ausüben. In einer bevorzugten Ausführungsform umfasst die Auswerteeinheit eine Steuereinheit für die Messeinheit und einen mit der Steuereinheit verbundenen Arbeitsplatzrechner. Die Auswertung kann zumindest teilweise bereits in der Messeinheit erfolgen.

Die Ausgabeeinheit ist z. B. ein Bildschirm, ein Berührungsbildschirm oder ein Drucker.

Die Messeinheit kann einen kapazitiven Sensor zur Erfassung einer Masse des ersten Prüfgutes und/oder einen optischen Sensor zur Erfassung einer Querdimension und/oder eines Fremdstoffgehaltes des ersten Prüfgutes beinhalten.

Die Erfindung ermöglicht einen Vergleich der Verläufe der ersten Dichtelinie und der Referenzdichtelinie. Aus dem Vergleich der Verläufe der Dichtelinien kann ein Vergleich der Qualitäten des ersten Prüfgutes und des Referenzprüfgutes vorgenommen werden.

Die Erfindung umfasst auch ein Verfahren zur Bereitstellung mindestens einer Referenzdichtelinie zur Verwendung im oben beschriebenen erfindungsgemässen Verfahren. Dabei wird eine für die weltweite Produktion repräsentative Menge von Referenzprüfgütern desselben Typs gesammelt. Messwerte mindestens einer Eigenschaft eines jeden Referenzprüfgutes werden entlang der Längsrichtung des Referenzprüfgutes erfasst. Werte mindestens eines Parameters des jeweiligen Referenzprüfgutes werden aus den Messwerten ermittelt. Ein Ereignisfeld wird bereitgestellt, das einen Quadranten oder einen Teil eines Quadranten eines zweidimensionalen kartesischen Koordinatensystems beinhaltet, dessen Abszisse eine Erstreckung von Parameterwerten in der Längsrichtung und dessen Ordinate eine Abweichung der Parameterwerte von einem Sollwert angibt, so dass die ermittelten Abweichungen der Parameterwerte und ihre Erstreckungen in der Längsrichtung als Ereignisse in dem Ereignisfeld eingetragen werden. Dichten der Ereignisse werden in dem Ereignisfeld ermittelt. In dem Ereignisfeld wird für jedes Referenzprüfgut eine Dichtelinie, welche im Wesentlichen einer konstanten, für jedes Referenzprüfgut desselben Typs gleichen Dichte der am Referenzprüfgut ermittelten Ereignisse folgt, ermittelt. In dem Ereignisfeld wird mindestens eine Perzentildichtelinie, welche ein Perzentil bezüglich der ermittelten Dichtelinien ist, als Referenzdichtelinie dargestellt. Vorteilhaft ist es, wenn die konstante Ereignisdichte zwischen 500 und 2000 Ereignissen pro 100 km Prüfgutlänge und vorzugsweise bei 1000 Ereignissen pro 100 km Prüfgutlänge liegt.

Für einen Typ von Referenzprüfgütern werden z. B. fünf Perzentildichtelinien, die dem 5-Perzentil, dem 25-Perzentil, dem 50-Perzentil, dem 75-Perzentil und dem 95-Perzentil entsprechen, als Referenzdichtelinien dargestellt.

Ferner umfasst die Erfindung ein Qualitäts-Referenzwerk, beinhaltend Qualitätsdaten für ein längliches Textiles Prüfgut. Das Qualitäts-Referenzwerk enthält mindestens eine Referenzdichtelinie, die nach dem oben beschriebenen Verfahren bereitgestellt wurde. Die mindestens eine Referenzdichtelinie aus einem solchen Qualitäts-Referenzwek kann dann im erfindungsgemässen Verfahren dazu verwendet werden, die Qualität des untersuchten Prüfgutes in Bezug auf die weltweite Produktion von Prüfgütern desselben Typs zu klassieren.

### AUFZÄHLUNG DER ZEICHNUNGEN

Nachfolgend wird die Erfindung anhand der schematischen Zeichnungen detailliert erläutert.
- Figur 1: zeigt schematisch eine erfindungsgemässe Vorrichtung.
- Figur 2: zeigt ein Ereignisfeld mit zwei Dichtelinien gemäss der Erfindung.
- Figur 3: zeigt ein Ereignisfeld mit drei Dichtelinien, und zusätzlich einem Prüfgutkörper, gemäss der Erfindung.
- Figur 4: zeigt ein Ereignisfeld mit Perzentilen zur Qualitätseinstufung eines Prüfgutes in Bezug auf die weltweite Garnproduktion.

### AUSFÜHRUNG DER ERFINDUNG

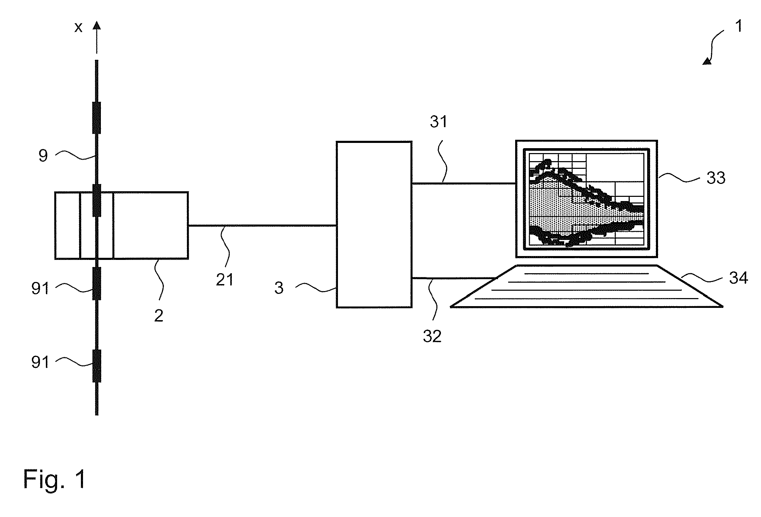

In **Figur 1** ist schematisch eine Vorrichtung 1 zur Durchführung des erfindungsgemässen Verfahrens dargestellt. Sie beinhaltet eine Messeinheit 2 zum Erfassen von Messwerten mindestens einer Eigenschaft eines ersten, entlang seiner Längsrichtung x bewegten länglichen textilen Prüfgutes 9, bspw. eines Garns. Derartige Messeinheiten 2 sind an sich bekannt und brauchen hier nicht näher erläutert zu werden. Die Messeinheit 2 kann z. B. als Garnreinigermesskopf ausgeführt sein und einen kapazitiven, optischen oder anderen Sensor beinhalten; es können auch mehrere gleiche oder verschiedene Sensoren innerhalb der Messeinheit 2 angeordnet sein. Im Fall eines kapazitiven Sensors sind die Messwerte bspw. eine Ausgangsspannung und/oder ein Ausgangsstrom des Sensors bzw. der entsprechenden Messschaltung, welche elektrischen Grössen ein Mass für die Dielektrizitätszahl des Prüfgutmaterials sind. Die Messeinheit 2 kann mit Auswertemitteln für eine Vorauswertung der Messdaten ausgestattet sein. Sie gibt auf einer ersten Datenleitung 21 ein vorzugsweise elektrisches Ausgangssignal aus, das ein Mass ist für die Masse, den Durchmesser oder andere Eigenschaften des ersten Prüfgutes 9.

Die erste Datenleitung 21 mündet in eine Auswerteeinheit 3, die zum Auswerten des Ausgangssignals der Messeinheit 2 eingerichtet ist. Zu diesem Zweck beinhaltet sie geeignete analoge und/oder digitale Auswertemittel, z. B. einen Mikroprozessor. Sie kann auch weitere Mittel wie Speichermittel zum Speichern von Daten beinhalten. Die Auswerteeinheit 3 ist vorzugsweise ein Computer.

Ferner beinhaltet die Vorrichtung 1 eine Ausgabeeinheit 33 zur Ausgabe von Messdaten und/oder Resultaten der Auswertung. Die Ausgabeeinheit 33 ist mittels einer zweiten Datenleitung 31 mit der Auswerteeinheit 3 verbunden. Sie kann z. B. als Bildschirm und/oder Drucker ausgebildet sein. Vorzugsweise beinhaltet die Vorrichtung 1 auch eine Eingabeeinheit 34 zum Eingeben von Daten durch einen Benutzer. Die Eingabeeinheit 34 ist mittels einer dritten Datenleitung 32 mit der Auswerteeinheit 3 verbunden und kann z. B. eine Tastatur oder eine Computermaus sein. Die Ausgabeeinheit 33 und die Eingabeeinheit 34 können in einem Berührungsbildschirm kombiniert sein.

Zwischen der Auswerteeinheit 3 und der Messeinheit 2 kann sich eine Steuereinheit befinden, die jedoch der Einfachheit halber in Figur 1 nicht eingezeichnet ist. Eine solche Steuereinheit dient dazu, die Messeinheit 2 einzustellen und zu steuern. Sie übernimmt auch teilweise die Auswertung der von der Messeinheit 2 erfassten Messwerte. Die nachfolgend beschriebenen Auswertungen können in der Messeinheit 2, in der Steuereinheit und/oder in der Auswerteeinheit 3 erfolgen.

Aus den erfassten Messwerten werden Werte mindestens eines Parameters des ersten Prüfgutes 9 ermittelt. Der Parameter kann bspw. die Masse pro Längeneinheit des ersten Prüfgutes 9 sein, die sich aus dem Ausgangssignal eines kapazitiven Sensors ergibt. Als Ereignisse 91 im ersten Prüfgut 9 werden Parameterwerte bezeichnet, die auf einer bestimmen Länge von einem Sollwert abweichen. Beispiele für solche Ereignisse 91 sind Dick- oder Dünnstellen, deren Masse pro Längeneinheit von einer Sollmasse pro Längeneinheit abweicht; bei einem Garn entspricht die Sollmasse pro Längeneinheit im Wesentlichen der Garnnummer.

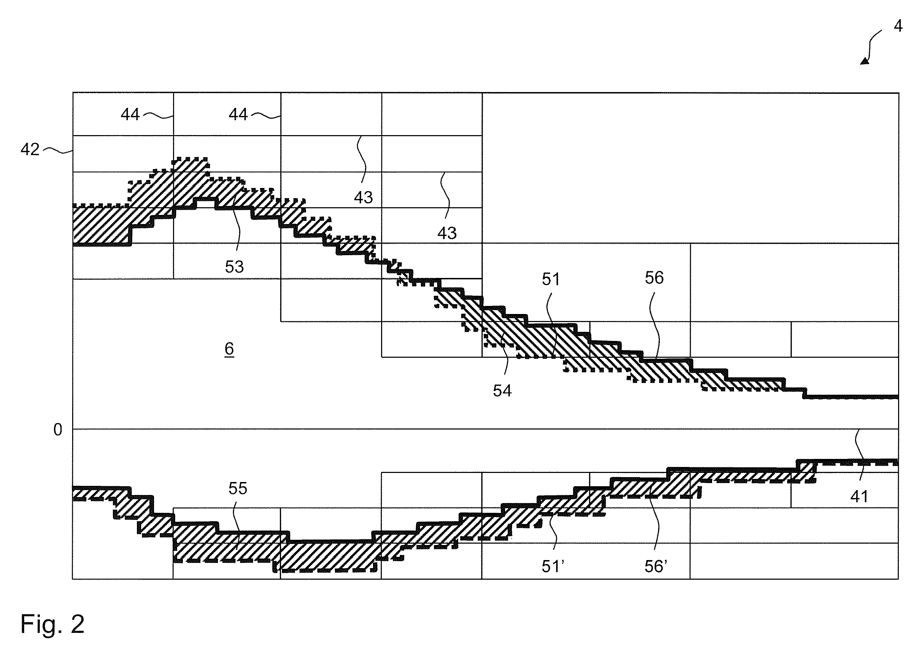

Es wird ein in **Figur 2** dargestelltes Ereignisfeld 4 bereitgestellt, das einen Quadranten oder einen Teil eines Quadranten eines zweidimensionalen kartesischen Koordinatensystems beinhaltet. Die Abszisse 41 des Koordinatensystems gibt eine Erstreckung von Parameterwerten in der Längsrichtung, d. h. eine Fehlerlänge, an; die Ordinate 42 gibt eine Abweichung des Parameters von einem Sollwert, d. h. eine Fehleramplitude, an. Der Parameter ist in diesem Beispiel die Masse pro Längeneinheit des ersten Prüfgutes 9. Zumindest ein Teil des Ereignisfeldes 4 ist durch waagrechte Klassengrenzen 43 und senkrechte Klassengrenzen 44 in rechteckige Klassen für Ereignisse unterteilt.

Ein genügend langer Abschnitt des ersten Prüfgutes 9 wird ausgemessen. Als "genügend" wird eine Messlänge von mindestens ca. 1 km angesehen; grössere Messlängen von z. B. 10 km oder 100 km Länge werden aber bevorzugt, weil sie statistisch aussagekräftigere Resultate liefern. Die Parameterwerte und die dazugehörigen Fehlerlängen werden von der Messeinheit 2 an die Auswerteeinheit 3 übermittelt. In der Auswerteeinheit 3 werden daraus Dichten von Ereignissen im Ereignisfeld 4 ermittelt, so wie es z. B. in der US-6,374,152 B1 beschrieben ist. Auf diese Weise kann jedem Punkt des Ereignisfeldes 4 eindeutig eine Ereignisdichte zugeordnet werden. Durch Interpolation, Extrapolation, Glättung und/oder andere numerische Verfahren können zu abrupte lokale Änderungen der so ermittelten Ereignisdichtefunktion, die möglicherweise durch Messfehler oder sonstige Artefakte verursacht sind, vermieden werden.

Es wird eine genügend hohe Ereignisdichte von z. B. 1000 Ereignissen pro 100 km Prüfgutlänge gewählt. Die Verbindung aller Punkte im Ereignisfeld 4, denen die Ereignisdichte zugeordnet ist, ergibt eine erste Dichtelinie 51, 51', welche im Wesentlichen der konstanten Ereignisdichte folgt. Die erste Dichtelinie 51, 51' ist für das untersuchte erste Prüfgut 9 charakteristisch. Die erste Dichtelinie 51 im oberen Teil von Figur 2 entspricht positiven Fehleramplituden, d. h. Nissen, kurzen Dickstellen und langen Dickstellen, während die erste Dichtelinie 51' im unteren Teil von Figur 2 negativen Fehleramplituden, d. h. Dünnstellen, entspricht. Das Ereignisfeld könnte alternativ nur die positiven oder nur die negativen Fehleramplituden beinhalten.

Gemäss dem erfindungsgemässen Verfahren wird im Ereignisfeld 4 zusätzlich zur ersten Dichtelinie 51, 51' eine Referenzdichtelinie 56, 56' dargestellt. Die Referenzdichtelinie 56, 56' folgt zwar derselben Ereignisdichte wie die erste Dichtelinie 51, 51' bezieht sich aber auf ein vom ersten Prüfgut 9 verschiedenes (nicht dargestelltes) Referenzprüfgut. Die Referenzdichtelinie 56, 56' und die erste Dichtelinie 51, 51' werden vorzugsweise unterschiedlich eingezeichnet, bspw. mit unterschiedlichen Strichsymbolen oder mit unterschiedlichen Farben.

Dank der gleichzeitigen Darstellung im Ereignisfeld 4 können die Verläufe der ersten Dichtelinie 51, 51' und der Referenzdichtelinie 56, 56' verglichen werden. Insbesondere interessieren Unterschiede zwischen den Verläufen der ersten Dichtelinie 51, 51' und der Referenzdichtelinie 56, 56'. Die Unterschiede können verdeutlicht werden, indem eine zumindest teilweise von der ersten Dichtelinie 51 (bzw. 51') und der Referenzdichtelinie 56 (bzw. 56') begrenzte Differenzfläche 53, 54 (bzw. 55) in dem Ereignisfeld 4 grafisch hervorgehoben, bspw. schraffiert oder eingefärbt, wird. Aus den festgestellten Unterschieden zwischen den Verläufen der ersten Dichtelinie 51, 51' und der Referenzdichtelinie 56, 56' wird auf Unterschiede zwischen den Qualitäten des ersten Prüfgutes 9 und des Referenzprüfgutes geschlossen. Im Beispiel von Figur 2 liegt im Bereich kleiner Fehlerlängen, d. h. der Nissen und der kurzen Fehler, die erste Dichtelinie 51 deutlich höher als die Referenzdichtelinie 56. Im Bereich grosser Fehlerlängen liegt die erste Dichtelinie 51 hingegen tiefer als die Referenzdichtelinie 56. Wenn sich im Beispiel von Figur 2 die Dichtelinien 51, 56 auf Garn beziehen, so kann aus dem Vergleich geschlossen werden, dass das erste Garn 9 im Gewebe ein eher unruhigeres, wolkiges Bild ergibt als das Referenzgarn, dafür weniger Streifen verursacht. Bezüglich der Dünnstellen im unteren Teil von Figur 2 (negative Fehleramplituden) ist das erste Garn 9 schlechter als das Referenzgarn.

Ausserdem ist im Ereignisfeld 4 von Figur 2 ein Referenzprüfgutkörper eingezeichnet, wie er an sich aus der WO-2010/078665 A1 bekannt ist. Der Referenzprüfgutkörper wird durch eine Fläche 6 dargestellt, welche durch die Ordinate 42 und die Referenzdichtelinie 56, 56' begrenzt ist. Die Form der den Referenzprüfgutkörper darstellenden Fläche 6 ist charakteristisch für das jeweilige Referenzprüfgut. Ereignisse, die im Referenzprüfgutkörper 6 liegen, gehören zum Referenzprüfgut und sollten nicht aus dem Referenzprüfgut entfernt werden.

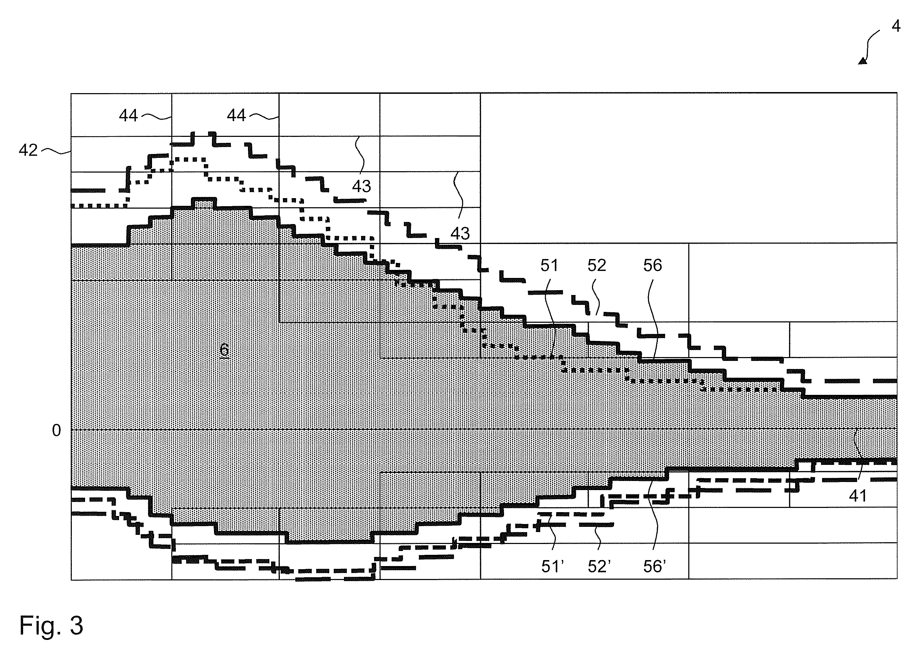

**Figur 3** zeigt ein Ereignisfeld 4, das demjenigen von Figur 2 ähnlich ist. Im Ereignisfeld 4 ist eine Referenzdichtelinie 56, 56' dargestellt. Die Fläche 6, die den Referenzprüfgutkörper darstellt, ist in diesem Ausführungsbeispiel durch einen Grauton hervorgehoben; alternativ könnte sie eine andere Farbe oder ein anderes Muster aufweisen als ihre Umgebung. Eine erste Dichtelinie 51, 51' ist für das untersuchte erste Prüfgut 9 charakteristisch. Zusätzlich zur ersten Dichtelinie 51, 51' ist in Figur 3 eine zweite Dichtelinie 52, 52' eingezeichnet, die sich auf ein zweites, vom ersten Prüfgut 9 verschiedenes Prüfgut bezieht. Zur besseren Unterscheidung und Identifizierung hat die zweite Dichtelinie 52, 52' vorzugsweise ein anderes Strichsymbol oder eine andere Farbe als die erste Dichtelinie 51, 51' und als die Referenzdichtelinie 56, 56'. Ein Vergleich zwischen der zweiten Dichtelinie 52, 52' und der Referenzdichtelinie 56, 56' zeigt, dass die Qualität des zweiten Prüfgutes schlechter ist als diejenige des Referenzprüfgutes. In analoger Weise können im Ereignisfeld 4 weitere (dritte, vierte, etc.) Prüfgutlinien, die sich auf weitere Prüfgüter beziehen, dargestellt und mit der Referenzdichtelinie 56, 56' verglichen werden.

Die Referenzdichtelinie 56, 56' kann auf verschiedene Arten ermittelt werden. Drei Arten werden im Sinne von Beispielen nachfolgend erläutert:
- Die Referenzdichtelinie 56, 56' kann auf dieselbe Weise wie die erste Dichtelinie 51, 51' ermittelt werden. Ein reales Referenzprüfgut wird entlang seiner Längsrichtung ausgemessen, Werte des mindestens einen Parameters werden aus den Messwerten ermittelt, Ereignisdichten werden aus den Parameterwerten ermittelt, und die Referenzdichtelinie 56, 56' folgt im Wesentlichen einer konstanten Dichte der am Referenzprüfgut ermittelten Ereignisse. Das Referenzprüfgut kann in diesem Fall z. B. eines von mehreren Prüfgütern 9 sein. Es kann einer Bedienungsperson ermöglicht werden, über die Eingabeeinheit 34 (Figur 1) einzugeben, welche von mehreren ermittelten Dichtelinien 51, 51' oder 52, 52' als Referenzdichtelinie 56, 56' verwendet werden soll.
- Wenn, wie im Beispiel von Figur 3, mehrere Prüfgüter ausgemessen werden, so kann die Referenzdichtelinie 56, 56' als Mittelwert aller ermittelten Dichtelinien 51, 51' und 52, 52' oder einer Teilmenge davon definiert werden. Bei der Mittelwertbildung kann eine Gewichtung vorgenommen werden, bspw. anhand der jeweils erfassten Länge des betreffenden Prüfgutes. In diesem Fall bezieht sich also die Referenzdichtelinie 56, 56' auf ein virtuelles Prüfgut, das aus einer Menge von mindestens zwei realen Prüfgütern gebildet wird.
- Schliesslich kann die Referenzdichtelinie 56, 56' in die Auswerteeinheit 3 eingegeben werden, ohne dass sie unmittelbar zuvor ermittelt worden wäre. Sie kann einem Qualitäts-Referenzwerk entnommen werden, wie z. B. den USTER^{®} *STATISTICS.* Alternativ kann die Referenzdichtelinie 56, 56' frei eingegeben oder zumindest verändert werden. Auch in diesem Fall bezieht sich die Referenzdichtelinie 56, 56' auf ein virtuelles Prüfgut, das aus einer Menge von mindestens zwei realen Prüfgütern gebildet wird.

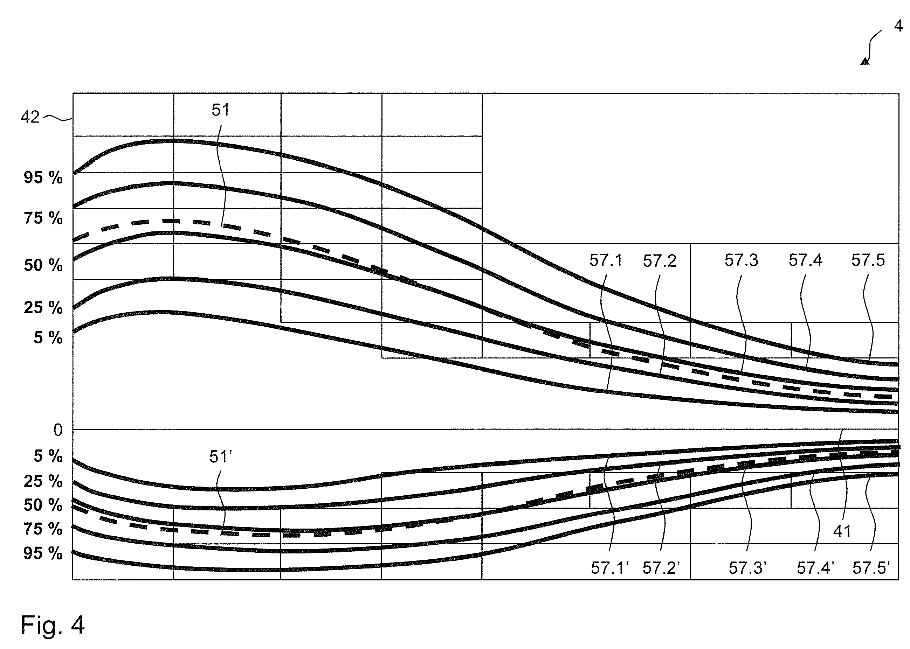

Die Referenzdichtelinie 56, 56' kann in ein Qualitäts-Referenzwerk wie die USTER^{®} *STATISTICS* aufgenommen werden. Zu diesem Zweck wird zunächst eine für die weltweite Prüfgutproduktion repräsentative Menge von Proben von Prüfgütern desselben Typs gesammelt, und ihre Dichtelinien werden berechnet. Bei Prüfgütern desselben Typs werden die Dichtelinien je nach Qualität des jeweiligen Prüfgutes in unterschiedlichen Höhen verlaufen, aber einander kaum schneiden. So können Perzentil-Dichtelinien 57.1-57.5, 57.1'-57.5' ermittelt werden, wie sie in **Figur 4** dargestellt sind. Die Perzentildichtelinien 57.1-57.5,57.1'-57.5' geben die 5-, 25-, 50-, 75- bzw. 95-Perzentile, bezogen auf die weltweite Prüfgutproduktion, an. Das heisst: 5 % aller weltweit produzierten Prüfgüter des betreffenden Typs haben Dichtelinien unterhalb (bzw. für Dünnstellen oberhalb) des 5-Perzentils 57.1, 25 % aller weltweit produzierten Prüfgüter haben Dichtelinien unterhalb (bzw. für Dünnstellen oberhalb) des 25-Perzentils 57.2, etc. Das 50-Perzentil 57.3, 57.3' gibt den weltweiten Median an.

Darstellungen wie das Ereignisfeld 4 von Figur 4 erlauben eine Klassierung der Qualität des Prüfgutes 9 in Bezug auf die weltweite Produktion von Prüfgut desselben Typs. Zu diesem Zweck wird eine oder mehrere der Perzentildichtelinien 57.1-57.5, 57.1'-57.5' als Referenzdichtelinie 56, 56' (siehe Figuren 2 und 3) im erfindungsgemässen Verfahren verwendet. Im Ereignisfeld 4 kann auch eine erste Dichtelinie 51, 51' dargestellt werden, die einem realen untersuchten Prüfgut 9 entspricht. Diese erste Dichtelinie 51, 51' wird mit den Perzentildichtelinien 57.1-57.5, 57.1'-57.5 verglichen, wobei eine Interpolation zwischen den diskreten Perzentildichtelinien 57.1-57.5, 57.1'-57.5 vorgenommen werden kann. Im Ausführungsbeispiel von Figur 4 führt ein solcher Vergleich etwa zu den folgenden Aussagen:
- Im Bereich der Nissen (sehr kurzen Dickstellen) entspricht die Lage der ersten Dichtelinie 51 dem 60-Perzentil.
- Im Bereich der kurzen Dickstellen entspricht die Lage der ersten Dichtelinie 51 dem 50-Perzentil, d. h. dem Median.
- Im Bereich langen Dickstellen entspricht die Lage der ersten Dichtelinie 51 dem 40-Perzentil.
- Für die Dünnstellen im unteren Teil des Ereignisfeldes liegt die erste Dichtelinie 51' in der Nähe des 50-Perzentils.
- Allgemein gesagt, handelt es sich um ein Prüfgut 9 mittlerer Qualität, verglichen mit der weltweiten Produktion.

Mit anderen Worten: Eine oder mehrere der Perzentildichtelinien 57.1-57.5, 57.1'-57.5' aus dem Ereignisfeld 4 von Figur 4 kann als Rererenzdichtelinie 56, 56' im Ereignisfeld 4 von Figur 2 oder 3 dargestellt und mit der ersten Dichtelinie 51, 51' (und möglicherweise weiteren Dichtelinien 52, 52') verglichen werden.

Selbstverständlich ist die vorliegende Erfindung nicht auf die oben diskutierten Ausführungsformen beschränkt. Bei Kenntnis der Erfindung wird der Fachmann weitere Varianten herleiten können, die auch zum Gegenstand der vorliegenden Erfindung gehören, wie sie in den unabhängigen Patentansprüchen definiert ist.

### BEZUGSZEICHENLISTE

- 1: Vorrichtung

- 2: Messeinheit
- 21: Datenleitung

- 3: Auswerteeinheit
- 31,32: Datenleitungen
- 33: Ausgabeeinheit
- 34: Eingabeeinheit

- 4: Ereignisfeld
- 41: Abszisse
- 42: Ordinate
- 43: waagrechte Klassengrenzen
- 44: senkrechte Klassengrenzen

- 51, 51': erste Dichtelinie
- 52, 52': zweite Dichtelinie
- 53-55: Differenzflächen
- 56, 56': Referenzdichtelinie
- 57.1-57.5, 57.1'-57.5': Perzentildichtelinien

- 6: Prüfgutkörper, Fläche

- 9: Prüfgut
- 91: Ereignisse

- x: Längs- und Bewegungsrichtung des Prüfgutes

## Patentansprüche

1. Verfahren zum Vergleich der Qualitäten von länglichen textilen Prüfgütern (9),
wobei
Messwerte mindestens einer Eigenschaft eines ersten Prüfgutes (9) entlang der Längsrichtung (x) des ersten Prüfgutes (9) erfasst werden,
Werte mindestens eines Parameters des ersten Prüfgutes (9) aus den Messwerten ermittelt werden,
ein Ereignisfeld (4) bereitgestellt wird, das einen Quadranten oder einen Teil eines Quadranten eines zweidimensionalen kartesischen Koordinatensystems beinhaltet, dessen Abszisse (41) eine Erstreckung von Parameterwerten in der Längsrichtung und dessen Ordinate (42) eine Abweichung der Parameterwerte von einem Sollwert angibt, so dass die ermittelten Abweichungen der Parameterwerte und ihre Erstreckungen in der Längsrichtung (x) als Ereignisse in dem Ereignisfeld (4) eingetragen werden,
Dichten der Ereignisse in dem Ereignisfeld (4) ermittelt werden und
in dem Ereignisfeld (4) eine erste Dichtelinie (51, 51'), welche im Wesentlichen einer konstanten Dichte der am ersten Prüfgut (9) ermittelten Ereignisse folgt, dargestellt wird,
**dadurch gekennzeichnet, dass**
in dem Ereignisfeld (4) zusätzlich zur ersten Dichtelinie (51, 51') eine Referenzdichtelinie (56, 56'), welche zwar derselben Ereignisdichte wie die erste Dichtelinie (51, 51') folgt, sich aber auf ein vom ersten Prüfgut (9) verschiedenes Referenzprüfgut bezieht, dargestellt wird, wobei
das Referenzprüfgut ein zweites, vom ersten Prüfgut (9) verschiedenes reales Prüfgut ist oder
das Referenzprüfgut ein virtuelles Prüfgut ist, das zum Zwecke der Bestimmung der Referenzdichtelinie (56, 56') aus einer Menge von mindestens zwei realen Prüfgütern gebildet wird.

2. Verfahren nach Anspruch 1, wobei das Referenzprüfgut ein virtuelles Prüfgut ist, das zum Zwecke der Bestimmung der Referenzdichtelinie (56, 56') aus einer Menge von mindestens zwei realen Prüfgütern gebildet wird, und die Menge nebst mindestens einem anderen realen Prüfgut auch das erste Prüfgut (9) beinhaltet.

3. Verfahren nach Anspruch 1 oder 2, wobei die Referenzdichtelinie einem Qualitäts-Referenzwerk entnommen wird.

4. Verfahren nach einem der vorangehenden Ansprüche, wobei eine zumindest teilweise von der ersten Dichtelinie (51, 51') und der Referenzdichtelinie (56, 56') begrenzte Differenzfläche (53-55) in dem Ereignisfeld (4) grafisch hervorgehoben wird.

5. Verfahren nach Anspruch 4, wobei sich die Differenzfläche (53-55) grafisch von ihrer Umgebung unterscheidet, insbesondere indem sie eine andere Farbe, einen anderen Grauton und/oder ein anderes Muster aufweist als ihre Umgebung.

6. Verfahren nach einem der vorangehenden Ansprüche, wobei in dem Ereignisfeld (4) ein Referenzprüfgutkörper als Fläche (6) dargestellt wird, die
einerseits durch die Abszisse (41) oder eine parallel dazu verlaufende Gerade, andererseits durch die Ordinate (42) oder eine parallel dazu verlaufende Gerade und
ferner durch die Referenzdichtelinie (56, 56')
begrenzt wird.

7. Verfahren nach Anspruch 6, wobei sich die den Referenzprüfgutkörper darstellende Fläche (6) grafisch von ihrer Umgebung unterscheidet, insbesondere indem sie eine andere Farbe, einen anderen Grauton und/oder ein anderes Muster aufweist als ihre Umgebung.

8. Verfahren nach einem der vorangehenden Ansprüche, wobei die konstante Ereignisdichte zwischen 500 und 2000 Ereignissen pro 100 km Prüfgutlänge und vorzugsweise bei 1000 Ereignissen pro 100 km Prüfgutlänge liegt.

9. Verfahren nach einem der vorangehenden Ansprüche, wobei in dem Ereignisfeld (4) zusätzlich zur sich auf das Referenzprüfgut beziehenden Referenzdichtelinie (56, 56') und zur sich auf das erste Prüfgut (9) beziehenden ersten Dichtelinie (51, 51') mindestens eine sich auf ein zweites Prüfgut beziehende zweite Dichtelinie (52, 52') dargestellt wird.

10. Verfahren nach einem der vorangehenden Ansprüche, wobei sich die erste Dichtelinie (51, 51'), die Referenzdichtelinie (56, 56') und allenfalls die zusätzlich dargestellte mindestens eine zweite Dichtelinie (52, 52') grafisch voneinander unterscheiden, insbesondere indem sie unterschiedliche Farben, unterschiedliche Grautöne, unterschiedliche Dicken und/oder unterschiedliche Strichsymbole aufweisen.

11. Vorrichtung (1) zum Vergleich der Qualität von länglichen textilen Prüfgütern (9), beinhaltend
eine Messeinheit (2) zur Erfassung von Messwerten mindestens einer Eigenschaft eines ersten Prüfgutes (9) entlang der Längsrichtung (x) des ersten Prüfgutes (9),
eine mit der Messeinheit (2) verbundene Auswerteeinheit (3), welche
für die Ermittlung von Werten mindestens eines Parameters des ersten Prüfgutes (9) aus den Messwerten,
für die Bereitstellung eines Ereignisfeldes (4), das einen Quadranten oder einen Teil eines Quadranten eines zweidimensionalen kartesischen Koordinatensystems beinhaltet, dessen Abszisse (41) eine Erstreckung von Parameterwerten in der Längsrichtung (x) und dessen Ordinate (42) eine Abweichung der Parameterwerte von einem Sollwert angibt, so dass die ermittelten Abweichungen der Parameterwerte und ihre Erstreckungen in der Längsrichtung (x) als Ereignisse in dem Ereignisfeld (4) eintragbar sind, und für die Ermittlung von Dichten der Ereignisse in dem Ereignisfeld (4)
eingerichtet ist, und
eine mit der Auswerteeinheit (3) verbundene Ausgabeeinheit (33) zur Darstellung des Ereignisfeldes (4) und einer ersten Dichtelinie (51, 51') in dem Ereignisfeld (4), welche im Wesentlichen einer konstanten Dichte der am ersten Prüfgut (9) ermittelten Ereignisse folgt,
**dadurch gekennzeichnet, dass**
die Auswerteeinheit (3) zur Speicherung einer Referenzdichtelinie (56, 56'), welche zwar derselben Ereignisdichte wie die erste Dichtelinie (51, 51') folgt, sich aber auf ein vom ersten Prüfgut (9) verschiedenes Referenzprüfgut bezieht, eingerichtet ist, wobei
das Referenzprüfgut ein zweites, vom ersten Prüfgut (9) verschiedenes reales Prüfgut ist oder
das Referenzprüfgut ein virtuelles Prüfgut ist, das zum Zwecke der Bestimmung der Referenzdichtelinie (56, 56') aus einer Menge von mindestens zwei realen Prüfgütern gebildet wird, und
die Ausgabeeinheit (33) zur gleichzeitigen Darstellung der ersten Dichtelinie (51, 51') und der Referenzdichtelinie (56, 56') eingerichtet ist.

12. Vorrichtung (1) nach Anspruch 11, wobei die Ausgabeeinheit (33) ein Bildschirm, ein Berührungsbildschirm oder ein Drucker ist.

13. Verfahren zur Bereitstellung mindestens einer Referenzdichtelinie (56, 56') zur Verwendung im Verfahren nach einem der Ansprüche 1-10, wobei eine für die weltweite Produktion repräsentative Menge von Referenzprüfgütern desselben Typs gesammelt wird,
Messwerte mindestens einer Eigenschaft eines jeden Referenzprüfgutes entlang der Längsrichtung des Referenzprüfgutes erfasst werden,
Werte mindestens eines Parameters des jeweiligen Referenzprüfgutes aus den Messwerten ermittelt werden,
ein Ereignisfeld (4) bereitgestellt wird, das einen Quadranten oder einen Teil eines Quadranten eines zweidimensionalen kartesischen Koordinatensystems beinhaltet, dessen Abszisse (41) eine Erstreckung von Parameterwerten in der Längsrichtung und dessen Ordinate (42) eine Abweichung der Parameterwerte von einem Sollwert angibt, so dass die ermittelten Abweichungen der Parameterwerte und ihre Erstreckungen in der Längsrichtung als Ereignisse in dem Ereignisfeld (4) eingetragen werden,
Dichten der Ereignisse in dem Ereignisfeld (4) ermittelt werden,
in dem Ereignisfeld (4) für jedes Referenzprüfgut eine Dichtelinie, welche im Wesentlichen einer konstanten, für jedes Referenzprüfgut desselben Typs gleichen Dichte der am Referenzprüfgut ermittelten Ereignisse folgt, ermittelt wird, und
in dem Ereignisfeld (4) mindestens eine Perzentildichtelinie (57.1-57.5, 57.1 '-57.5'), welche ein Perzentil bezüglich der ermittelten Dichtelinien ist, als Referenzdichtelinie (56, 56') dargestellt wird.

14. Verfahren nach Anspruch 13, wobei die konstante Ereignisdichte zwischen 500 und 2000 Ereignissen pro 100 km Prüfgutlänge und vorzugsweise bei 1000 Ereignissen pro 100 km Prüfgutlänge liegt.

15. Qualitäts-Referenzwerk, beinhaltend Qualitätsdaten für ein längliches textiles Prüfgut,
**dadurch gekennzeichnet, dass**
das Qualitäts-Referenzwerk mindestens eine Referenzdichtelinie (56, 56') enthält, die nach dem Verfahren gemäss Anspruch 13 oder 14 bereitgestellt wurde.

## Claims

1. A method for comparing the qualities of elongated textile test materials (9), wherein measured values of at least one property of a first test material (9) are detected along the longitudinal direction (x) of the first test material (9),
values of at least one parameter of the first test material (9) are determined from the measured values,
an event field (4) is provided which contains a quadrant or a part of a quadrant of a two-dimensional Cartesian coordinate system, whose abscissa (41) indicates an extension of parameter values in the longitudinal direction and whose ordinate (42) indicates a deviation of the parameter values from a target value, so that the determined parameter values and their extensions in the longitudinal direction (x) care entered as events in the event field (4),
densities of the events in the event field (4) are determined, and
a first density line (51,51'), which substantially follows a constant density of the events determined in the first test material (9), is represented in the event field (4),
**characterized in that**
a reference density line (56, 56'), which follows the same event density as the first density line (51,51') but relates to a reference test material that differs from the first test material (9), is represented in the event field (4) in addition to the first density line (51, 51'), wherein
the reference test material is a second real test material which differs from the first test material (9) or
the reference test material is a virtual test material which is formed for the purpose of determining the reference density line (56, 56') from a set of at least two real test materials.

2. The method according to claim 1, wherein the reference test material is a virtual test material which is formed for the purpose of determining the reference density line (56, 56') from a set of at least two real test materials and the set also contains the first test material (9) in addition to at least one other real test material.

3. The method according to claim 1 or 2, wherein the reference density line is taken from a quality reference document.

4. The method according to one of the preceding claims, wherein a differential area (53 to 55), which is bounded at least partly by the first density line (51,51') and the reference density line (56, 56'), is graphically emphasized in the event field (4).

5. The method according to claim 4, wherein the differential area (53 to 55) graphically differs from its surrounding area, especially in that it has a different color, a different grey shade and/or a different pattern than its surrounding area.

6. The method according to one of the preceding claims, wherein a reference test material body is represented as an area (6) in the event field (4), which area is bounded
by the abscissa (41) or a straight line extending parallel thereto on the one hand, by the ordinate (42) or a straight line extending parallel thereto on the other hand, and
further by the reference density line (56, 56').

7. The method according to claim 6, wherein the area (6) representative of the reference test material body differs graphically from its surrounding area, especially in that it has a different color, a different grey shade and/or a different pattern than its surrounding area.

8. The method according to one of the preceding claims, wherein the constant event density lies between 500 and 2000 events per 100 km of test material length and preferably at 1000 events per 100 km of test material length.

9. The method according to one of the preceding claims, wherein at least one second density line (52, 52') that relates to a second test material is represented in the event field (4) in addition to the reference density line (56, 56') relating to the reference test material and to the first density line (51,51') relating to the first test material (9).

10. The method according to one of the preceding claims, wherein the first density line (51,51'), the reference density line (56, 56') and optionally the additionally represented, at least one second density line (52, 52') graphically differ from each other, especially in that they have different colors, different grey shades, different thicknesses and/or different dash symbols.

11. An apparatus (1) for comparing the quality of elongated textile test materials (9), containing
a measuring unit (2) for detecting measured values of at least one property of a first test material (9) along the longitudinal direction (x) of the first test material (9), and an evaluation unit (3) connected to the measuring unit (2) which is set up
for determining values of at least one parameter of the first test material (9) from the measured values,
for the provision of an event field (4) which contains a quadrant or a part of a quadrant of a two-dimensional Cartesian coordinate system, whose abscissa (41) indicates an extension of parameter values in the longitudinal direction (x) and whose ordinate (42) indicates a deviation of the parameter values from a target value, so that the determined parameter values and their extensions in the longitudinal direction (x) can be entered as events in the event field (4), and for the determination of densities of the events in the event field (4), and
an output unit (33) connected to the evaluation unit (3) for displaying the event field (4) and a first density line (51, 51') in the event field (4), which substantially follows a constant density of the events determined on the first test material (9), **characterized in that**
the evaluation unit (3) is set up for storing a reference density line (56, 56'), which follows the same event density as the first density line (51,51') but relates at least in part to a reference test material which differs from the first test material (9), wherein the reference test material is a second real test material which differs from the first test material (9) or
the reference test material is a virtual test material which is formed for the purpose of determining the reference density line (56, 56') from a set of at least two real test materials, and
the output unit (33) is set up for the simultaneous representation of the first density line (51, 51') and the reference density line (56, 56').

12. The apparatus (1) according to claim 11, wherein the output unit (33) is a screen, a touchscreen or a printer.

13. A method for providing at least one reference density line (56, 56') for use in the method according to one of the claims 1 to 10, wherein
a set of reference test materials of the same type which is representative for the worldwide production is collected,
measured values of at least one property of each reference test material are detected along the longitudinal direction of the reference test material,
values of at least one parameter of the respective reference test material are determined from the measured values,
an event field (4) is provided which contains a quadrant or a part of a quadrant of a two-dimensional Cartesian coordinate system, whose abscissa (41) indicates an extension of parameter values in the longitudinal direction and whose ordinate (42) indicates a deviation of the parameter values from a target value, so that the determined parameter values and their extensions in the longitudinal direction are entered as events in the event field (4),
densities of the events in the event field (4) are determined,
a density line, which substantially follows a constant density of the events determined on the first test material, which density is the same for each reference test material of the same type, is determined in the event field (4) for each reference test material, and
at least one percentile density line (57.1-57.5, 57.1'-57.5'), which is a percentile concerning the determined density lines, is represented in the event field (4) as a reference density line (56, 56').

14. The method according to claim 20, wherein the constant event density lies between 500 and 2000 events per 100 km of test material length and preferably at 1000 events per 100 km of test material length.

15. A quality reference document, containing quality data for an elongated textile test material, **characterized in that**
the quality reference document contains at least one reference density line (56, 56') which was provided according to the method according to one of the claims 13 to 14.

## Revendications

1. Procédé pour comparer les qualités d'articles textiles allongés à contrôler (9), dans lequel
des valeurs de mesure d'au moins une propriété d'un premier article à contrôler (9) sont acquises dans le sens de la longueur (x) du premier article à contrôler (9),
des valeurs d'au moins un paramètre du premier article à contrôler (9) sont déterminées à partir des valeurs de mesure,
un champ d'événements (4) est créé, qui contient un quadrant ou une partie de quadrant d'un système de coordonnées cartésiennes à deux dimensions dont l'abscisse (41) indique une étendue de valeurs de paramètre dans le sens de la longueur et l'ordonnée (42) un écart des valeurs de paramètre par rapport à une valeur de consigne, de sorte que les écarts déterminés des valeurs de paramètre et leurs étendues dans le sens de la longueur (x) sont saisis sous forme d'événements dans le champ d'événements (4),
des densités des événements dans le champ d'événements (4) sont déterminées et
une première ligne de densité (51, 51'), qui suit pour l'essentiel une densité constante des événements déterminés dans le premier article à contrôler (9), est représentée dans le champ d'événements (4),
**caractérisé en ce que**
outre la première ligne de densité (51,51'), une ligne de densité de référence (56, 56') est représentée dans le champ d'événements (4), laquelle suit la même densité d'événements que la première ligne de densité (51, 51') mais fait référence à un article à contrôler de référence différent du premier article à contrôler (9),
l'article à contrôler de référence étant un deuxième article à contrôler réel différent du premier article à contrôler (9) ou
l'article à contrôler de référence étant un article à contrôler virtuel créé aux fins de la détermination de la ligne de densité de référence (56, 56') à partir d'un groupe d'au moins deux articles de référence réels.

2. Procédé selon la revendication 1, dans lequel l'article à contrôler de référence est un article à contrôler virtuel créé aux fins de la détermination de la ligne de densité de référence (56, 56') à partir d'un groupe d'au moins deux articles de référence réels et le groupe inclut, outre au moins un autre article à contrôler réel, le premier article à contrôler (9).

3. Procédé selon la revendication 1 ou 2, dans lequel la ligne de densité de référence est tirée d'un document de référence de qualité.

4. Procédé selon l'une des revendications précédentes, dans lequel une surface de différence (53-55) au moins, partiellement délimitée par la première ligne de densité (51, 51') et la ligne de densité de référence (56, 56'), est mise en évidence graphiquement dans le champ d'événements (4).

5. Procédé selon la revendication 4, dans lequel la surface de différence (53-55) se différencie graphiquement de son environnement, en particulier par une autre couleur, un autre ton de gris et/ou un autre motif que son environnement.

6. Procédé selon l'une des revendications précédentes, dans lequel un corps d'article à contrôler de référence est représenté dans le champ d'événements (4) sous la forme d'une aire (6) qui est délimitée,
d'une part, par l'abscisse (41) ou une droite parallèle à celle-ci, et
d'autre part, par l'ordonnée (42) ou une droite parallèle à celle-ci,
ainsi que par la ligne de densité de référence (56, 56').

7. Procédé selon la revendication 6, dans lequel l'aire (6) représentant le corps d'article à contrôler de référence se différencie graphiquement de son environnement, en particulier par une autre couleur, un autre ton de gris et/ou un autre motif que son environnement.

8. Procédé selon l'une des revendications précédentes, dans lequel la densité d'événements constante se situe entre 500 et 2000 événements pour 100 km de longueur d'article à contrôler et de préférence à 1000 événements pour 100 km de longueur d'article à contrôler.

9. Procédé selon l'une des revendications précédentes, dans lequel, en plus de la ligne de densité de référence (56, 56') se rapportant à l'article à contrôler de référence et de la première ligne de densité (51, 51') se rapportant au premier article à contrôler (9), au moins une deuxième ligne de densité (52, 52') se rapportant à un deuxième article à contrôler est représentée dans le champ d'événements (4).

10. Procédé selon l'une des revendications précédentes, dans lequel la première ligne de densité (51, 51'), la ligne de densité de référence (56, 56') et éventuellement l'au moins une deuxième ligne de densité (52, 52') représentée en plus se différencient les unes des autres graphiquement, en particulier par des couleurs différentes, des tons de gris différents, des épaisseurs différentes et/ou des symboles de traits différents.

11. Dispositif (1) pour comparer la qualité d'articles textiles allongés à contrôler (9), comprenant
une unité de mesure (2) destinée à acquérir des valeurs de mesure d'au moins une propriété d'un premier article à contrôler (9) dans le sens de la longueur (x) du premier article à contrôler (9),
une unité d'analyse (3) reliée à l'unité de mesure (2), qui est équipée
pour déterminer des valeurs d'au moins un paramètre du premier article à contrôler (9) à partir des valeurs de mesure,
pour produire un champ d'événements (4) qui contient un quadrant ou une partie de quadrant d'un système de coordonnées cartésiennes à deux dimensions dont l'abscisse (41) indique une étendue de valeurs de paramètre dans le sens de la longueur (x) et l'ordonnée (42) indique un écart des valeurs de paramètre par rapport à une valeur de consigne, de sorte que les écarts déterminés des valeurs de paramètre et leurs étendues dans le sens de la longueur (x) sont saisis sous forme d'événements dans le champ d'événements (4), et
pour déterminer des densités des événements dans le champ d'événements (4),
et
une unité de sortie (33) reliée à l'unité d'analyse (3) et destinée à représenter le champ d'événements (4) et une première ligne de densité (51, 51') dans le champ d'événements (4), qui suit pour l'essentiel une densité constante des événements déterminés dans le premier article à contrôler (9),
**caractérisé en ce que**
l'unité d'analyse (3) est équipée pour enregistrer une ligne de densité de référence (56, 56') qui présente la même densité d'événements que la première ligne de densité (51, 51') mais se rapporte à un article à contrôler de référence différent du premier article à contrôler (9), l'article à contrôler de référence étant un deuxième article à contrôler réel différent du premier article à contrôler (9) ou
l'article à contrôler de référence étant un article à contrôler virtuel créé aux fins de la détermination de la ligne de densité de référence (56, 56') à partir d'un groupe d'au moins deux articles de référence réels, et
l'unité de sortie (33) est équipée pour représenter simultanément la première ligne de densité (51, 51') et de la ligne de densité de référence (56, 56').

12. Dispositif (1) selon la revendication 11, dans lequel l'unité de sortie (33) est un écran, un écran tactile ou une imprimante.

13. Procédé pour la production d'au moins une ligne de densité de référence (56, 56') à utiliser dans le procédé selon l'une des revendications 1 à 10, dans lequel un groupe d'articles à contrôler de référence du même type représentatif de la production mondiale est recueilli,
des valeurs de mesure d'au moins une propriété de chaque article à contrôler de référence sont acquises dans le sens de la longueur de l'article à contrôler de référence,
des valeurs d'au moins un paramètre de l'article à contrôler de référence en question sont déterminées à partir des valeurs de mesure,
un champ d'événements (4) est produit, qui contient un quadrant ou une partie de quadrant d'un système de coordonnées cartésiennes à deux dimensions dont l'abscisse (41) indique une étendue de valeurs de paramètre dans le sens de la longueur et l'ordonnée (42) indique un écart des valeurs de paramètre par rapport à une valeur de consigne, de sorte que les écarts déterminés des valeurs de paramètre et leurs étendues dans le sens de la longueur sont saisis sous forme d'événements dans le champ d'événements (4),
des densités des événements du champ d'événements (4) sont déterminées,
une ligne de densité, suivant pour l'essentiel une densité des événements déterminés dans l'article à contrôler de référence constante et identique pour chaque article à contrôler de référence, est déterminée dans le champ d'événements (4), et
au moins une ligne de densité de percentile (57.1-57.5, 57.1'-57.5'), qui est un percentile par rapport aux lignes de densité déterminées, est représentée dans le champ d'événements (4) comme ligne de densité de référence (56, 56').

14. Procédé selon la revendication 13, dans lequel dans lequel la densité d'événements constante se situe entre 500 et 2000 événements pour 100 km de longueur d'article à contrôler et de préférence à 1000 événements pour 100 km de longueur d'article à contrôler.

15. Document de référence de qualité contenant des données de qualité pour un article textile allongé à contrôler, **caractérisée en ce que** le document de référence de qualité contient au moins une ligne de densité de référence (56, 56') produite selon le procédé selon la revendication 13 ou 14.
